# EUROPEAN PATENT SPECIFICATION

(11) **EP 0 709 094 B1**
(45) Date of publication and mention of the grant of the patent: **05.04.2000**
(21) Application number: 95307739.3
(22) Date of filing: 30.10.1995
(51) Int. Cl.: A61K 31/44, A61K 31/445, A61K 31/535

(54) **Tetrahydropyridine oxadiazole or thiadiazole compound for treating anxiety**
Tetrahydropyridin Oxadiazol- oder -Thiadiazol-Verbindung zur Behandlung von Angstzuständen
Tétrahydropyridine oxadiazole ou thiadiazole pour traiter l'anxiété

(30) Priority: 31.10.1994 US 332186; 09.11.1994 US 336454
(43) Date of publication of application: 01.05.1996
(73) Proprietor: ELI LILLY AND COMPANY, Indianapolis, Indiana 46285 (US)
(72) Inventor: Bodick, Neil C., Indianapolis, Indiana 46260 (US); Bymaster, Franklin P., Brownsburg, Indiana 46112 (US); Offen, Walter W., Indianapolis, Indiana 46236 (US); Shannon, Harlan E., Carmel, Indiana 46285 (US)
(74) Representative: Hudson, Christopher Mark

(56) References cited:
- EP-A- 0 384 288
- WO-A-94/20495
- WO-A-95/05174
- JOURNAL OF MEDICINAL CHEMISTRY, vol. 35, no. 12, 1992 pages 2274-2283, P. SAUERBERG ET AL. 'NOVEL FUNCTIONAL M1 SELECTIVE MUSCARINIC AGONISTS'

## Description

Extensive research has been conducted for a number of years directed toward the development of compounds capable of treating anxiety in humans that are safer to the user and which exhibit fewer side-effects. For example, several clinically established anxiolytic agents such as the barbiturates, meprobamate and the benzodiazepines have numerous side effects such as potential for abuse and addiction or potentiation of the effects of ethanol. The mechanism of action of these compounds is believed to involve the GABA/benzodiazepine receptor complex in humans.

Buspirone is another compound which has been studied for the treatment of anxiety. The literature states that Buspirone interacts with reasonable potency only at the 5-HT_{1A} and dopamine receptors. Alfred Goodman, *et al.*, Goodman and Gilman's The pharmacological Basis of Therapeutics, **8**:482 (1990); Tompkins *et al.* Research Communications in Psychology, Psychiatry, and Behavior, **5**:4, p. 338 (1980).

Sauerberg *et al.* in U.S. Patents 5,043,345, 5,041,455 and 5,260314 disclose the compounds employed in the present invention as cholinergic compounds. As such, the compounds are taught to be useful in treating Alzheimer's disease, severe painful conditions, and glaucoma. There is no disclosure in the patents of using the compounds to treat anxiety.

The art has reported that compounds which act as agonists of the cholinergic muscarinic receptor can actually produce anxiety. See, Risch *et al.* Psychopharmacol. Bull., **19**: 696-698 (1983), Nurnberger *et al*. Psychiatry Res., **9**:191-200 (1983), and Nurnberger *et al*. Psychopharmacol. Bull., **17**:80-82 (1982).

Surprisingly, we have discovered that a group of compounds having muscarinic cholinergic activity can be useful for treating anxiety. The present invention relates to the treatment of anxiety. More specifically, the invention provides the use of a tetrahydropyridine or azabicyclic oxadiazole or thiadiazole compound for the manufacture of a medicament for treating anxiety in humans. The activity of these compounds is believed to be based on agonist action at the m-1 muscarinic cholinergic receptor. As noted hereinbefore, the compounds employed in the method of the present invention are known. Methods of preparing the compounds, as well as pharmaceutical formulations containing the compounds, are taught by Sauerberg in U.S. Pat. Nos. 5,041,455, 5,043,345, and 5,260,314. Certain tetrahydropyridine compound useful as stimulants of cognitive function are disclosed in EP-A-384 288.

The present invention provides the use of a compound of Formula I: 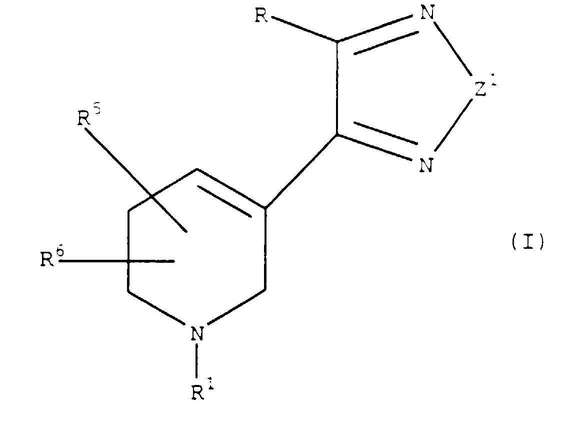 wherein
Z1 is oxygen or sulphur;
R is hydrogen, halogen, amino, -NHCO-R², C₃₋₇-cycloalkyl, C₄₋₁₀-(cycloalkylalkyl), -Z²⁻C₃₋₇-cycloalkyl optionally substituted with C₁₋₆-alkyl, -Z²-C₄₋₁₀-(cycloalkylalkyl), -Z²-C₄₋₁₀-(cycloalkenylalkyl), -Z²-C₄₋₁₀-(methylenecycloalkyl-alkyl), -NH-R2, -NR2R3, -NH-OR2, phenyl, phenoxy, benzoyl, benzyloxycarbonyl, tetrahydronaphtyl, indenyl, X, R², -Z²R², -SOR², -SO₂R², -Z²-R²-Z³-R³, -Z²-R²-Z³-R³-Z⁴-R⁴, -Z²-R²CO-R³, -Z²-R²-CO₂-R³, Z²-R²-O₂C-R³, -Z²-R²-CONH-R³, -Z²-R²-NHCOR³, -Z²-R²-X, -Z²-R²-Z³-X, wherein Z², Z³, and Z⁴ independently are oxygen or sulphur, and R², R³ and R⁴ independently are straight or branched C₁₋₁₅-Alkyl, straight or branched C₂₋₁₅-alkenyl, straight or branched C₂₋₁₅-alkynyl, each of which is optionally substituted with halogen(s), -OH, -CN, -CF₃, -SH, -COOH, -NH-R², -NR²R³, C₁₋₆alkyl ester, one or two phenyl, phenoxy, benzoyl or benzyloxycarbonyl wherein each aromatic group is optionally substituted with one or two halogen, -CN, C₁₋₄-alkyl or C₁₋₄-alkoxy, and X is a 5 or 6 membered heterocyclic group containing one to four N, 0 or S atom(s) or a combination thereof, which heterocyclic group is optionally substituted at carbon or nitrogen atom(s) with straight or branched C₁₋₆-alkyl, phenyl, benzyl or pyridine, or a carbon atom in the heterocyclic group together with an oxygen atom form a carbonyl group, or which heterocyclic group is optionally fused with a phenyl group; and
R⁵ and R⁶ may be present at any position, including the point of attachment of the thiadiazole or oxadiazole ring, and independently are hydrogen, straight or branched C₁₋₅-alkyl, straight or branched C₂₋₅-alkenyl, straight or branched C₂₋₅-alkynyl, straight or branched C₁₋₁₀-alkoxy, straight or branched C₁₋₅-alkyl substituted with -OH, halogen, -NH₂ or carboxy; R¹ is hydrogen, straight or branched C₁₋₅-alkyl, straight or branched C₂₋₅-alkenyl or straight or branched C₂₋₅-alkynyl; or
a pharmaceutically acceptable salt or solvate thereof for the manufacture of a medicament for the treatment of anxiety.

It is to be understood that the invention extends to the use of each of the stereoisomeric forms of the compounds of the present invention as well as the pure diastereomeric, pure enantiomeric, and racemic forms of the named compounds.

The term "antianxiety dose", as used herein, represents an amount of compound necessary to prevent or treat a human susceptible to or suffering from anxiety following administration to such human. The active compounds are effective over a wide dosage range. For example, dosages per day will normally fall within the range of about 0.005 to about 500 mg/kg of body weight. In the treatment of adult humans, the range of about 0.05 to about 100 mg/kg, in single or divided doses, is preferred. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration. While the present compounds are preferably administered orally to humans susceptible to or suffering from anxiety, the compounds may also be administered by a variety of other routes such as the transdermal, parenterally, subcutaneous, intranasal, intramuscular and intravenous routes. Such formulations may be designed to provide delayed or controlled release using formulation techniques which are known in the art.

As used herein the term "treating" includes prophylaxis of a physical and/or mental condition or amelioration or elimination of the developed physical and/or mental condition once it has been established or alleviation of the characteristic symptoms of such condition.

As used herein the term "anxiety" refers to an anxiety disorder. Examples of anxiety disorders which may preferably be treated using an effective amount of a named compound or pharmaceutically acceptable salt thereof include, but are not limited to: Panic Attack; Agoraphobia; Acute Stress Disorder; Specific Phobia; Panic Disorder; Psychoactive Substance Anxiety Disorder; Organic Anxiety Disorder; Obsessive-Compulsive Anxiety Disorder; Posttraumatic Stress Disorder; Generalized Anxiety Disorder; and Anxiety Disorder. Not Otherwise Specified (NOS).

Examples of anxiety disorders which may more preferably be treated using an effective amount of a named compound or a pharmaceutically acceptable salt thereof include Panic Attack; Panic Disorder; Psychoactive Substance Anxiety Disorder; Organic Anxiety Disorder; Obsessive-Compulsive Anxiety Disorder; Posttraumatic Stress Disorder; Generalized Anxiety Disorder; and Anxiety Disorder NOS.

Examples of the anxiety disorders which are most preferably treated using a named compound include Organic Anxiety Disorder; Obsessive-Compulsive Disorder; Posttraumatic Stress Disorder; Generalized Anxiety Disorder; and Anxiety Disorder NOS.

The named anxiety disorders have been characterized in the DSM-IV-R. Diagnostic and Statistical Manual of Mental Disorders, Revised, 4th Ed. (1994). The DSM-IV-R was prepared by the Task Force on Nomenclature and Statistics of the American Psychiatric Association, and provides clear descriptions of diagnostic catagories. The skilled artisan will recognize that there are alternative nomenclatures, nosologies, and classification systems for pathologic psychological conditions and that these systems evolve with medical scientific progress.

The compounds employed in the invention are not believed to act via the GABA/benzodiazepine, 5HT1A, or D1 receptor systems in humans. Rather, the activity of the present compounds as antianxiety agents is believed to be based upon modulation of muscarinic cholinergic receptors. However, the mechanism by which the present compounds function is not necessarily the mechanism stated *supra.,* and the present invention is not limited by any mode of operation.

The following Examples are studies to establish the usefulness of the named compounds for treating anxiety.

### Example 1

### Punished Responding

The antianxiety activity of the compounds employed in the use of the present invention is established by demonstrating that the compounds increase punished responding. This procedure has been used to establish antianxiety activity in clinically established compounds.

According to this procedure, the responding of rats or pigeons is maintained by a multiple schedule of food presentation. In one component of the schedule, responding produces food pellet presentation only. In a second component, responding produces both food pellet presentation and is also punished by presentation of a brief electric shock. Each component of the multiple schedule is approximately 4 minutes in duration, and the shock duration is approximately 0.3 seconds. The shock intensity is adjusted for each individual animal so that the rate of punished responding is approximately 15 to 30% of the rate in the unpunished component of the multiple schedule. Sessions are conducted each weekday and are approximately 60 min in duration. Vehicle or a dose of compound are administered 30 min to 6 hr before the start of the test session by the subcutaneous or oral route. Compound effects for each dose for each animal are calculated as a percent of the vehicle control data for that animal. The data are expressed as the mean ± the standard error of the mean.

### Example 2

### Monkey Taming Model

Further, the antianxiety activity of the compounds is established by demonstrating that the compounds are effective in the monkey taming model. Plotnikoff Res. Comm. Chem. Path. & Pharmacol., **5**: 128-134 (1973) described the response of rhesus monkeys to pole prodding as a method of evaluating the antiaggressive activity of a test compound. In this method, the antiaggressive activity of a compound was considered to be indicative of its antianxiety activity. Hypoactivity and ataxia were considered to be indicative of a sedative component of the compound. The present study is designed to measure the pole prod response-inhibition induced by a compound of this invention in comparison with that of a standard antianxiety compound such as diazepam as a measure of antiaggressive potential, and to obtain an indication of the duration of action of the compound.

Male and female rhesus or cynomologous monkeys, selected for their aggressiveness toward a pole, are housed individually in a primate colony room. Compounds or appropriate vehicle are administered orally or subcutaneously and the animals are observed by a trained observer at varying times after drug administration. A minimum of three days (usually a week or more) elapses between treatments. Treatments are assigned in random fashion except that no monkey receives the same compound two times consecutively.

Aggressiveness and motor impairment are graded by response to a pole being introduced into the cage as described in Table 1. The individuals responsible for grading the responses are unaware of the dose levels received by the monkeys.

**Table 1**

| Grading of Monkey Response to Pole Introduction | | |
|---|---|---|
| Response | Grade | Description |
| Attack | **2** | Monkey immediately grabbed and/or bit pole as it was placed at opening in cage. |
| | **1** | Monkey grabbed and/or bit pole only after the tip was extended into the cage 30 cm (12 inches) or more. |
| | **0** | No grabbing or biting observed. |
| Pole Push | **2** | Monkey grabbed the pole to attack it or push it away. |
| | **1** | Monkey touched the pole only in attempting to avoid it or rode on the pole (avoidance). |
| | **0** | No pushing, grabbing or riding of the pole observed. |
| Biting | **2** | Monkey bit aggressively and frequently. |
| | **1** | Monkey bit weakly or infrequently |
| | **0** | No biting observed. |
| | | |
| Ataxia | **2** | Monkey exhibited a marked loss of coordination. |
| | **1** | Slight loss of coordination observed. |
| | **0** | No effects on coordination observed. |
| Hypoactivity | **2** | Marked: Monkey was observed in a prone position. May or may not have responded by rising and moving away when experimenter approached. |
| | **1** | Slight: Monkey did not retreat as readily when experimenter approached |
| | **0** | None. |
| Antiaggression | **+** | Dose of drug was active in decreasing global assessment of aggressive behavior |
| Activity of Drug Dose | **-** | Dose of drug was not active in decreasing aggressive behavior. |

### Example 3

### Human Clinical Trials

Finally, the antianxiety activity of the named compounds can be demonstrated by human clinical trials. The study was designed as a double-blind, parallel, placebo-controlled multicenter trial. The patients were randomized into four groups, placebo and 25, 50, and 75 mg tid (three times a day) of test compound. The dosages were administered orally with food. Patients were observed at four visits to provide baseline measurements. Visits 5-33 served as the treatment phase for the study.

During the visits, patients and their caregivers were questioned and observed for signs of agitation, mood swings, vocal outbursts, suspiciousness, and fearfulness. Each of these behaviors are indicative of the effect of the test compound on an anxiety disorder.
For example, one test compound produced the following results:

| | **Placebo** (N=87) | | **25 mg** (N=85) | | **50 mg** (N=83) | | **75 mg** (N=87) | | **p-Value** |
|---|---|---|---|---|---|---|---|---|---|
| Behavioral Event | n | (%) | n | (%) | n | (%) | n | (%) | |
| Agitation | 40 | (46) | 34 | (40) | 24 | (29) | 20 | (23) | .006 |
| Mood swings | 40 | (46) | 25 | (29) | 21 | (25) | 28 | (32) | .025 |
| Vocal Outbursts | 33 | (38) | 29 | (34) | 24 | (29) | 11 | (13) | .001 |
| Suspiciousness | 32 | (37) | 23 | (27) | 26 | (31) | 7 | (8) | <.001 |
| Fearfulness | 25 | (29) | 28 | (33) | 19 | (23) | 13 | (15) | .038 |

Treatment groups were compared with respect to the number and percent of patients who ever had the symptom during the double-blind portion of the study (visits 5 through 33), at a severity that was worse than during the baseline visits (1 through 4).

Preferred compounds for use in treating anxiety include:
1,2,5,6-Tetrahydro-3-(3-methoxy-1,2,5-thiadiazol-4-yl)-1-methylpyridine;
3-(3-Ethoxy-1,2,5-thiadiazol-4-yI)-1,2,5,6-tetrahydro-1-methylpyridine;
1,2,5,6-Tetrahydro-1-methyl-3-(3-propoxy-1,2,5-thiadiazol-4-yl)pyridine;
3-(3-Butoxy-1,2,5-thiadiazol-4-yl)-1,2,5,6-tetrahydro-1-methylpyridine;
1,2,5,6-Tetrahydro-3-(3-isopropoxy-1,2,5-thiadiazol-4-yl)-1-methylpyridine;
1,2,5,6-Tetrahydro-1-methyl-3-(3-pentyloxy-1,2,5-thiadiazol-4-yl)pyridine;
1,2,5,6-Tetrahydro-3-(3-isobutoxy-1,2,5-thiadiazol-4-yl)-1-methylpyridine;
1,2,5,6-Tetrahydro-3-(3-isopentyloxy-1,2,5-thiadiazol-4-yl)-1-methylpyridine;
3-(3-Hexyloxy-1,2,5-thiadiazol-4-yl)-1,2,5,6-tetrahydro-1-methylpyridine;
3-(3-Benzyloxy-1,2,5-thiadiazol-4-yl)-1,2,5,6-tetrahydro-1-methylpyridine;
3-(3-(3-Butenyloxy)-1,2,5-thiadiazol-4-yl)-1,2,5,6-tetrahydro-1-methylpyridine;
3-(3-(2-Butynyloxy)-1,2,5-thiadiazol-4-yl)-1,2,5,6-tetrahydro-1-methylpyridine;
1,2,5,6-Tetrahydro-1-methyl-3-(3-propargyloxy-1,2,5-thiadiazol-4-yl)pyridine;
3-(3-Cyclopropylmethoxy-1,2,5-thiadiazol-4-yl)-1,2,5,6-tetrahydro-1-methylpyridine;
1,2,5,6-Tetrahydro-3-(3-methoxyethoxy-1,2,5-thiadiazol-4-yl)-1-methylpyridine;
3-(3-Chloro-1,2,5-thiadiazol-4-yl)-1,2,5,6-tetrahydro-1-methylpyridine;
3-(3-Chloro-1,2,5-thiadiazol-4-yl)-1,2,5,6-tetrahydropyridine;
3-(3-Butoxy-1,2,5-thiadiazol-4-yl)-1,2,5,6-tetrahydropyridine;
3-(3-Chloro-1,2,5-thiadiazol-4-yl)-1-ethyl-1,2,5,6-tetrahydropyridine;
3-(3-Ethoxy-1,2,5-thiadiazol-4-yl)-1-ethyl-1,2,5,6-tetrahydropyridine;
3-(3-Heptyloxy-1,2,5-thiadiazol-4-yl)-1,2,5,6-tetrahydro-1-methylpyridine;
3-(3-(3-Pentynyloxy)-1,2,5-thiadiazol-4-yl)-1,2,5,6-tetrahydro-1-methylpyridine;
3-(3-(4-Pentenyloxy)-1,2,5-thiadiazol-4-yl)-1,2,5,6-tetrahydro-1-methylpyridine;
3-(3-(2-Propenyloxy)-1,2,5-thiadiazol-4-yl)-1,2,5,6-tetrahydro-1-methylpyridine;
3-(3-Octyloxy-1,2,5-thiadiazol-4-yl)-1,2,5,6-tetrahydro-1-methylpyridine;
3-(3-(3-Hexynyloxy)-1,2,5-thiadiazol-4-yl)-1,2,5,6-tetrahydro-1-methylpyridine;
3-(3-(3-Methyl-2-butenyloxy)-1,2,5-thiadiazol-4-yl)-1,2,5,6-tetrahydro-1-methylpyridine;
3-(3-(3-Butenyl-2-oxy)-1,2,5-thiadiazol-4-yl)-1,2,5,6-tetrahydro-1-methylpyridine;
3-(3-(4-Hexenyloxy)-1,2,5-thiadiazol-4-yl)-1,2,5,6-tetrahydro-1-methylpyridine;
trans-3-(3-(3-Hexenyloxy)-1,2,5-thiadiazol-4-yl)-1,2,5,6-tetrahydro-1-methylpyridine;
cis-3-(3-(2-Pentenyloxy)-1,2,5-thiadiazol-4-yl)-1,2,5,6-tetrahydro-1-methylpyridine;
cis-3-(3-(2-Hexenyloxy)-1,2,5-thiadiazol-4-yl)-1,2,5,6-tetrahydro-1-methylpyridine;
3-(3-(5-Hexenyloxy)-1,2,5-thiadiazol-4-yl)-1,2,5,6-tetrahydro-1-methylpyridine;
cis-3-(3-(3-Hexenyloxy)-1,2,5-thiadiazol-4-yl)-1,2,5,6-tetrahydro-1-methylpyridine;
trans-3-(3-(2-Hexenyloxy)-1,2,5-thiadiazol-4-yl)-1,2,5,6-tetrahydro-1-methylpyridine;
3-(1,2,5-Thiadiazol-3-yl)-1,2,5,6-tetrahydro-1-methylpyridine;
1,2,5,6-Tetrahydro-3-(3-hexyloxy-1,2,5-thiadiazol-4-yl)pyridine;
3-(3-(2-(2-Methoxyethoxy)ethoxy)-1,2,5-thiadiazol-4-yl)-1,2,5,6-tetrahydro-1-methylpyridine;
3-(3-(3-Ethoxy-1-propoxy)-1,2,5-thiadiazol-4-yl)-1,2,5,6-tetrahydro-1-methylpyridine;
3-(3-(2-Ethoxyethoxy)-1,2,5-thiadiazol-4-yl)-1,2,5,6-tetrahydro-1-methylpyridine;
3-(3-(2-Butoxyethoxy)-1,2,5-thiadiazol-4-yl)-1,2,5,6-tetrahydro-1-methylpyridine;
3-(3-(2-(2-Butoxyethoxy)ethoxy)-1,2,5-thiadiazol-4-yl)-1,2,5,6-tetrahydro-1-methylpyridine;
3-(3-(2-(2-Ethoxyethoxy)ethoxy)-1,2,5-thiadiazol-4-yl)-1,2,5,6-tetrahydro-1-methylpyridine;
3-(3-(4-Methylpiperidino)-1,2,5-thiadiazol-4-yl)-1,2,5,6-tetrahydro-1-methylpyridine;
3-(3-Morpholino-1,2,5-thiadiazol-4-yl)-1,2,5,6-tetrahydro-1-methylpyridine;
3-(3-Hexylamino-1,2,5-thiadiazol-4-yl)-1,2,5,6-tetrahydro-1-methylpyridine;
3-(3-Propylthio-1,2,5-thiadiazol-4-yl)-1,2,5,6-tetrahydro-1-methylpyridine;
3-(3-Butylthio-1,2,5-thiadiazol-4-yl)-1,2,5,6-tetrahydro-1-methylpyridine;
3-(3-Methylthio-1,2,5-thiadiazol-4-yl)-1,2,5,6-tetrahydro-1-methylpyridine;
3-(3-Amino-1,2,5-oxadiazol-4-yl)-1,2,5,6-tetrahydro-1-methylpyridine;
3-(3-Acetylamino-1,2,5-oxadiazol-4-yl)-1,2,5,6-tetrahydro-1-methylpyridine;
3-(3-Chloro-1,2,5-oxadiazol-4-yl)-1,2,5,6-tetrahydro-1-methylpyridine;
3-(1,2,5-Oxadiazol-3-yl)-1,2,5,6-tetrahydro-1-methylpyridine;
3-(3-Hexyloxy-1,2,5-oxadiazol-4-yl)-1,2,5,6-tetrahydro-1-methylpyridine;
3-(3-Butyloxy-1,2,5-oxadiazol-4-yl)-1,2,5,6-tetrahydro-1-methylpyridine;
3-(3-(3-Hexynyloxy)-1,2,5-oxadiazol-4-yl)-1,2,5,6-tetrahydro-1-methylpyridine;
3-(3-Pentyl-1,2,5-thiadiazol-4-yl)-1,2,5,6-tetrahydro-1-methylpyridine;
3-(3-Heptyl-1,2,5-thiadiazol-4-yl)-1,2,5,6-tetrahydro-1-methylpyridine;
3-(3-(5-Hexenyl)-1,2,5-thiadiazol-4-yl)-1,2,5,6-tetrahydro-1-methylpyridine;
3-(3-Octyl-1,2,5-thiadiazol-4-yl)-1,2,5,6-tetrahydro-1-methylpyridine;
3-(3-lsobutyl-1,2,5-thiadiazol-4-yl)-1,2,5,6-tetrahydro-1-methylpyridine;
3-(3-Cyclopropylmethyl-1,2,5-thiadiazol-4-yl)-1,2,5,6-tetrahydro-1-methylpyridine;
3-(3-Propyl-1,2,5-thiadiazol-4-yl)-1,2,5,6-tetrahydro-1-methylpyridine;
3-(3-Octylthio-1,2,5-thiadiazol-4-yl)-1,2,5,6-tetrahydro-1-methylpyridine;
3-(3-Ethylthio-1,2,5-thiadiazol-4-yl)-1,2,5,6-tetrahydro-1-methylpyridine;
3-(3-Pentylthio-1,2,5-thiadiazol-4-yl)-1,2,5,6-tetrahydro-1-methylpyridine;
3-(3-Hexylthio-1,2,5-thiadiazol-4-yl)-1,2,5,6-tetrahydro-1-methylpyridine;
3-(3-(5-Cyanopentylthio)-1,2,5-thiadiazol-4-yl)-1,2,5,6-tetrahydro-1-methylpyridine;
3-(3-(3-Chloropropylthio)-1,2,5-thiadiazol-4-yl)-1,2,5,6-tetrahydro-1-methylpyridine;
3-(3-(3-Cyanopropylthio)-1,2,5-thiadiazol-4-yl)-1,2,5,6-tetrahydro-1-methylpyridine;
3-(3-(3-Phenylpropylthio)-1,2,5-thiadiazol-4-yl)-1,2,5,6-tetrahydro-1-methylpyridine;
3-(3-(2-Phenoxyethylthio)-1,2,5-thiadiazol-4-yl)-1,2,5,6-tetrahydro-1-methylpyridine;
3-(3-(4-Cyanobutylthio)-1,2,5-thiadiazol-4-yl)-1,2,5,6-tetrahydro-1-methylpyridine;
3-(3-(8-Hydroxyoctylthio)-1,2,5-thiadiazol-4-yl)-1,2,5,6-tetrahydro-1-methylpyridine;
3-(3-(4-Chlorobutylthio)-1,2,5-thiadiazol-4-yl)-1,2,5,6-tetrahydro-1-methylpyridine;
3-(3-(4,4-Bis-(4-fluorophenyl)-butylthio)-1,2,5-thiadiazol-4-yl)-1,2,5,6-tetrahydro-1-methylpyridine;
3-(3-(4-Cyanobenzylthio)-1,2,5-thiadiazol-4-yl)-1,2,5,6-tetrahydro-1-methylpyridine;
3-(3-(2-Phenylethylthio)-1,2,5-thiadiazol-4-yl)-1,2,5,6-tetrahydro-1-methylpyridine;
3-(3-(4-Bromobenzylthio)-1,2,5-thiadiazol-4-yl)-1,2,5,6-tetrahydro-1-methylpyridine;
3-(3-(4-Methylbenzylthio)-1,2,5-thiadiazol-4-yl)-1,2,5,6-tetrahydro-1-methylpyridine;
3-(3-(2-Benzoylethylthio)-1,2,5-thiadiazol-4-yl)-1,2,5,6-tetrahydro-1-methylpyridine;
3-(3-(4-Oxo-4-(4-fluorophenyl)-butylthio)-1,2,5-thiadiazol-4-yl)-1,2,5,6-tetrahydro-1-methylpyridine;
3-(3-Benzyloxycarbonylmethylthio-1,2,5-thiadiazol-4-yl)-1,2,5,6-tetrahydro-1-methylpyridine;
3-(3-Benzylthio-1,2,5-thiadiazol-4-yl)-1,2,5,6-tetrahydro-1-methylpyridine;
3-(3-(4,4,4-Trifluorobutylthio)-1,2,5-thiadiazol-4-yl)-1,2,5,6-tetrahydro-1-methylpyridine;
3-(3-(5,5,5-Trifluoropentylthio)-1,2,5-thiadiazol-4-yl)-1,2,5,6-tetrahydro-1-methylpyridine;
3-(3-(6,6,6-Trifluorohexylthio)-1,2,5-thiadiazol-4-yl)-1,2,5,6-tetrahydro-1-methylpyridine;
3-(3-Ethoxycarbonylpentylthio-1,2,5-thiadiazol-4-yl)-1,2,5,6-tetrahydro-1-methylpyridine;
1,2,5,6-Tetrahydro-1-methyl-3-(3-(6,6,6-trifluorohexyloxy)-1,2,5-thiadiazol-4-yl)pyridine;
1,2,5,6-Tetrahydro-1-methyl-3-(3-(3-(4-methoxyphenyl)-1-propoxy)-1,2,5-thiadiazol-4-yl)pyridine;
1,2,5,6-Tetrahydro-1-methyl-3-(3-(2-(4-methoxyphenyl)-1-ethoxy)-1,2,5-thiadiazol-4-yl)pyridine;
1,2,5,6-Tetrahydro-1-methyl-3-(3-(3-hydroxy-1-propoxy)-1,2,5-thiadiazol-4-yl)pyridine;
1,2,5,6-Tetrahydro-1-methyl-3-(3-(2-phenyl-1-ethoxy)-1,2,5-thiadiazol-4-yl)pyridine;
1,2,5,6-Tetrahydro-1-methyl-3-(3-(3-hydroxy-1-hexyloxy)-1,2,5-thiadiazol-4-yl)pyridine;
1,2,5,6-Tetrahydro-1-methyl-3-(3-(3-phenyl-1-propoxy)-1,2,5-thiadiazol-4-yl)pyridine;
1,2,5,6-Tetrahydro-1-methyl-3-(3-(6-acetamido-1-hexyloxy)-1,2,5-thiadiazol-4-yl)pyridine;
1,2,5,6-Tetrahydro-1-methyl-3-(3-(2-acetamido-1-ethoxy)-1,2,5-thiadiazol-4-yl)pyridine;
1,2,5,6-Tetrahydro-1-methyl-3-(3-(2-propionamido-1-ethoxy)-1,2,5-thiadiazol-4-yl)pyridine;
1,2,5,6-Tetrahydro-1-methyl-3-(3-(2-benzylthio-1-ethoxy)-1,2,5-thiadiazol-4-yl)pyridine;
1,2,5,6-Tetrahydro-1-methyl-3-(3-(2-ureido-1-ethoxy)-1,2,5-thiadiazol-4-yl)pyridine;
1,2,5,6-Tetrahydro-1-methyl-3-(3-(2-ethylsulfinyl-1-ethoxy)-1,2,5-thiadiazol-4-yl)pyridine;
1,2,5,6-Tetrahydro-3-(3-(5-oxohexyl)-1,2,5-thiadiazol-4-yl)-1-methylpyridine;
3-(3-(3-Phenylpropylthio)-1,2,5-oxadiazol-4-yl)-1,2,5,6-tetrahydro-1-methylpyridine;
3-(3-(2-Phenoxyethylthio)-1,2,5-oxadiazol-4-yl)-1,2,5,6-tetrahydro-1-methylpyridine;
3-(3-(2-(1,3-Dioxolane-2-yl)-ethylthio)-1,2,5-thiadiazol-4-yl)-1,2,5,6-tetrahydro-1-methylpyridine;
3-(3-(4-Pyridylmethylthio)-1,2,5-thiadiazol-4-yl)-1,2,5,6-tetrahydro-1-methylpyridine;
1,2,5,6-Tetrahydro-1-methyl-3-(3-(3-(2-thienyl)-1-propoxy)-1,2,5-thiadiazol-4-yl)pyridine;
1,2,5,6-Tetrahydro-1-methyl-3-(3-(2-(2-thienyl)-1-ethoxy)-1,2,5-thiadiazol-4-yl)pyridine;
1,2,5,6-Tetrahydro-1-methyl-3-(3-(2-(3-thienyl)-1-ethoxy)-1,2,5-thiadiazol-4-yl)pyridine;
1,2,5,6-Tetrahydro-1-methyl-3-(3-(2-thienylmethoxy)-1,2,5-thiadiazol-4-yl)pyridine;
1,2,5,6-Tetrahydro-1-methyl-3-(3-(3-thienylmethoxy)-1,2,5-thiadiazol-4-yl)pyridine;
1,2,5,6-Tetrahydro-1-methyl-3-(3-(3-(2-pyrrolidon-1-yl)-1-propoxy)-1,2,5-thiadiazol-4-yl)pyridine;
1,2,5,6-Tetrahydro-1-methyl-3-(3-(2-(2-pyrrolidon-1-yl)-1-ethoxy)-1,2,5-thiadiazol-4-yl)pyridine;
1,2,5,6-Tetrahydro-1-methyl-3-(3-(2-(2-oxazolidon-3-yl)-1-ethoxy)-1,2,5-thiadiazol-4-yl)pyridine;
1,2,5,6-Tetrahydro-1-methyl-3-(3-(3-(1-pyrrolidyl)-1-propoxy)-1,2,5-thiadiazol-4-yl)pyridine;
1-(3-(3-Pyridyl)-1,2,5-thiadiazol-4-ylthio)-4-(3-(1-methyl-1,2,5,6-tetrahydropyridin-3-yl)-1,2,5-thiadiazol-4-ylthio)butane;
1-(1-Methyltetrazol-5-ylthio)-4-(3-(1-methyl-1,2,5,6-tetrahydropyridin-3-yl)-1,2,5-thiadiazol-4-ylthio)butane;
1-(2-Methyl-1,3,4-thiadiazol-5-ylthio)-4-(3-(1-methyl-1,2,5,6-tetrahydropyridin-3-yl)-1,2,5-thiadiazol-4-ylthio)butane;
1-(2-Thiazolin-2-ylthio)-4-(3-(1-methyl-1,2,5,6-tetrahydropyridin-3-yl)-1,2,5-thiadiazol-4-ylthio)butane;
1-(2-Benzoxazolylthio)-4-(3-(1-methyl-1,2,5,6-tetrahydropyridin-3-yl)-1,2,5-thiadiazol-4-ylthio)butane;
1-(2-Methyl-1,3,4-thiadiazol-5-ylthio)-5-(3-(1-methyl-1,2,5,6-tetrahydropyridin-3-yl)-1,2,5-thiadiazol-4-ylthio)pentane;
1-(2-Benzthiazolylthio)-5-(3-(1-methyl-1,2,5,6-tetrahydropyridin-3-yl)-1,2,5-thiadiazol-4-ylthio)pentane;
1-(1-Methyltetrazol-5-ylthio)-5-(3-(1-methyl-1,2,5,6-tetrahydropyridin-3-yl)-1,2,5-thiadiazol-4-ylthio) pentane;
1-(2-Methyl-1,3,4-thiadiazol-5-ylthio)-6-(3-(1-methyl-1,2,5,6-tetrahydropyridin-3-yl)-1,2,5-thiadiazol-4-ylthio)hexane;
1-(1-Methyltetrazol-5-ylthio)-6-(3-(1-methyl-1,2,5,6-tetrahydropyridin-3-yl)-1,2,5-thiadiazol-4-ylthio)hexane;
1-(2-Thiazolin-2-ylthio)-6-(3-(1-methyl-1,2,5,6-tetrahydropyridin-3-yl)-1,2,5-thiadiazol-4-ylthio)hexane;
3-(3-Methylsulfonyl-1,2,5-thiadiazol-4-yl)-1,2,5,6-tetrahydro-1-methylpyridine;
3-(3-[2-(1-Pyrrolidinyl)ethoxy]-1,2,5-thiadiazol-4-yl)-1,2,5,6-tetrahydro-1-methylpyridine;
3-(3-(3-(5-Methyl-2-thienyl)-1-propoxy)-1,2,5-thiadiazol-4-yl)-1,2,5,6-tetrahydro-1-methylpyridine;
3-(3-((5-Propyl-2-thienyl)methoxy)-1,2,5-thiadiazol-4-yl)-1,2,5,6-tetrahydro-1-methylpyridine;
3-(3-(3-(5-Pentyl-2-thienyl)-1-propoxy)-1,2,5-thiadiazol-4-yl)-1,2,5,6-tetrahydro-1-methylpyridine;
3-(3-(3-(2-Thienylthio)-1-propoxy)-1,2,5-thiadiazol-4-yl)-1,2,5,6-tetrahydro-1-methylpyridine;
3-(3-(3-(2-Thienyl)-1-propylthio)-1,2,5-thiadiazol-4-yl)-1,2,5,6-tetrahydro-1-methylpyridine;
3-(3-(2-Thienylmethylthio)-1,2,5-thiadiazol-4-yl)-1,2,5,6-tetrahydro-1-methylpyridine;
3-(3-(3-(2-Oxazolidinon-3-yl)-1-propylthio)-1,2,5-thiadiazol-4-yl)-1,2,5,6-tetrahydro-1-methylpyridine;
3-(3-(3-(2-Thiazolidinon-3-yl)-1-propylthio-1,2,5-thiadiazol-4-yl)-1,2,5,6-tetrahydro-1-methylpyridine;
3-(3-(5-Pentyl-2-thienyl)methylthio-1,2,5-thiadiazol-4-yl)-1,2,5,6-tetrahydro-1-methylpyridine;
(R)-(+) 3-(3-(3-(4-Benzyl-2-oxazolidinon-3-yl)-1-propylthio)-1,2,5-thiadiazol-4-yl)-1,2,5,6-tetrahydro-1-methylpyridine;
(S)-(-)3-(3-(3-(4-Benzyl-2-oxazolidinon-3-yl)-1-propylthio)-1,2,5-thiadiazol-4-yl)-1,2,5,6-tetrahydro-1-methylpyridine;
(4R,5S)-3-(3-(3-(4-Methyl-5-phenyl-2-oxazolidinon-3-yl)-1-propylthio)-1,2,5-thiadiazol-4-yl)-1,2,5,6-tetrahydro-1-methylpyridine;
(S)-3-(3-(3-(4-Isopropyl-2-oxazolidinon-3-yl)-1-propylthio)-1,2,5-thiadiazol-4-yl)-1,2,5,6-tetrahydro-1-methylpyridine;
(S)-3-(3-(3-(4-Ethyl-2-oxazolidinon-3-yl)-1-propylthio)-1,2,5-thiadiazol-4-yl)-1,2,5,6-tetrahydro-1-methylpyridine;
(S)-3-(3-(3-(4-(2-Butyl)-2-oxazolidinon-3-yl)-1-propylthio-1,2,5-thiadiazol-4-yl)-1,2,5,6-tetrahydro-1-methylpyridine;
3-(3-(3-(4-Propyl-2-oxazolidinon-3-yl)-1-propylthio)-1,2,5-thiadiazol-4-yl)-1,2,5,6-tetrahydro-1-methylpyridine;
1,2,5,6-Tetrahydro-3-(3-methoxy-1,2,5-thiadiazol-4-yl)-1,4-dimethylpyridine;
3-(3-Hexyloxy-1,2,5-thiadiazol-4-yl)-1,2,5,6-tetrahydro-1,4-dimethylpyridine;
3-(3-Hexylthio-1,2,5-thiadiazol-4-yl)-1,2,5,6-tetrahydro-1,6-dimethylpyridine;
3-(3-Pentylthio-1,2,5-thiadiazol-4-yl)-1,2,5,6-tetrahydro-1,6-dimethylpyridine;
3-(3-(4-Cyanobenzylthio)-1,2,5-thiadiazol-4-yl)-1,2,5,6-tetrahydro-1,6-dimethylpyridine;
3-(3-(4-Cyanobutylthio)-1,2,5-thiadiazol-4-yl)-1,2,5,6-tetrahydro-1,6-dimethylpyridine;
3-(3-Butylthio-1,2,5-thiadiazol-4-yl)-1,2,5,6-tetrahydro-1,6-dimethylpyridine;
3-(3-Ethylthio-1,2,5-thiadiazol-4-yl)-1,2,5,6-tetrahydro-1,6-dimethylpyridine;
3-(3-(4-Pentynylthio)-1,2,5-thiadiazol-4-yl)-1,2,5,6-tetrahydro-1,6-dimethylpyridine;
3-(3-(3-Phenylpropylthio)-1,2,5-thiadiazol-4-yl)-1,2,5,6,tetrahydro-1,6-dimethylpyridine;
3-(3-Hexyloxy-1,2,5-thiadiazol-4-yl)-1,2,5,6-tetrahydro-1,6-dimethylpyridine;
3-(3-Pentyloxy-1,2,5-thiadiazol-4-yl)-1,2,5,6-tetrahydro-1,6-dimethylpyridine
3-(3-Butoxy-1,2,5-thiadiazol-4-yl)-1,2,5,6-tetrahydro-1,6-dimethylpyridine;
3-(3-(4-Pentenyloxy)-1,2,5-thiadiazol-4-yl)-1,2,5,6-tetrahydro-1,6-dimethylpyridine;
3-(3-(3-Hexynyloxy)-1,2,5-thiadiazol-4-yl)-1,2,5,6-tetrahydro-1,6-dimethylpyridine;
3-(3-Ethoxy-1,2,5-thiadiazol-4-yl)-1,2,5,6-tetrahydro-1,6-dimethylpyridine;
3-(3-(2,4-Dimethylphenylpropoxy)-1,2,5-thiadiazol-4-yl)-1,2,5,6-tetrahydro-1-methylpyridine;
3-(3-(3,4-Dimethylphenylpropoxy)-1,2,5-thiadiazol-4-yl)-1,2,5,6-tetrahydro-1-methylpyridine;
3-(3-(5-Ethyl-2-thienylmethoxy)-1,2,5-thiadiazol-4-yl)-1,2,5,6-tetrahydro-1-methylpyridine;
3-(3-(Pyrrolidin-1-yl)propoxy-1,2,5-thiadiazol-4-yl)-1,2,5,6-tetrahydro-1-methylpyridine;
3-(3-(4-Fluorophenylpropoxy)-1,2,5-thiadiazol-4-yl)-1,2,5,6-tetrahydro-1-methylpyridine;
3-(3-(4-Chlorophenylpropoxy)-1,2,5-thiadiazol-4-yl)-1,2,5,6-tetrahydro-1-methylpyridine;
3-(3-(3-Methylphenylpropoxy)-1,2,5-thiadiazol-4-yl)-1,2,5,6-tetrahydro-1-methylpyridine;
3-(3-(2,3-Dihydro-1-indenyloxy)-1,2,5-thiadiazol-4-yl)-1,2,5,6-tetrahydro-1-methylpyridine;
3-(3-(4-Methylphenylpropoxy)-1,2,5-thiadiazol-4-yl)-1,2,5,6-tetrahydro-1-methylpyridine;
3-(3-(1,2,3,4-Tetrahydro-2-naphtalyloxy)-1,2,5-thiadiazol-4-yl)-1,2,5,6-tetrahydro-1-methylpyridine;
3-(3-Phenylbutoxy-1,2,5-thiadiazol-4-yl)-1,2,5,6-tetrahydro-1-methylpyridine;
3-(3-(2-Methylphenylpropoxy)-1,2,5-thiadiazol-4-yl)-1,2,5,6-tetrahydro-1-methylpyridine;
3-(3-(2,5-Dimethylphenylpropoxy)-1,2,5-thiadiazol-4-yl)-1,2,5,6-tetrahydro-1-methylpyridine;
3-(3-Methylthioethoxy-1,2,5-thiadiazol-4-yl)-1,2,5,6-tetrahydro-1-methylpyridine;
3-(3-Dimethylaminoethoxy-1,2,5-thiadiazol-4-yl)-1,2,5,6-tetrahydro-1-methylpyridine;
3-(3-(3,4-Dichlorophenylpropoxy)-1,2,5-thiadiazol-4-yl)-1,2,5,6-tetrahydro-1-methylpyridine;
3-(3-Dimethylaminopropoxy-1,2,5-thiadiazol-4-yl)-1,2,5,6-tetrahydro-1-methylpyridine;
3-(3-(4-Ethylbenzyloxy)-1,2,5-thiadiazol-4-yl)-1,2,5,6-tetrahydro-1-methylpyridine;
3-(3-(4-Methylphenylpropoxy)-1,2,5-thiadiazol-4-yl)-1,2,5,6-tetrahydro-1-methylpyridine;
3-(3-(4-Butylbenzyloxy)-1,2,5-thiadiazol-4-yl)-1,2,5,6-tetrahydro-1-methylpyridine;
3-(3-(1-Ethylpentyloxy)-1,2,5-thiadiazol-4-yl)-1,2,5,6-tetrahydro-1-methylpyridine;
3-(3-(1-Ethylbutoxy)-1,2,5-thiadiazol-4-yl)-1,2,5,6-tetrahydro-1-methylpyridine;
3-(3-(1-Methylpentyloxy)-1,2,5-thiadiazol-4-yl)-1,2,5,6-tetrahydro-1-methylpyridine;
3-(3-(5-Hexynyloxy)-1,2,5-thiadiazol-4-yl)-1,2,5,6-tetrahydro-1-methylpyridine;
3-(3-(3-(4-Cyclohexylbutoxy)-1,2,5-thiadiazol-4-yl)-1,2,5,6-tetrahydro-1-methylpyridine;
3-(3-(5-Hydroxyhexyloxy)-1,2,5-thiadiazol-4-yl)-1,2,5,6-tetrahydro-1-methylpyridine;
3-(3-(5-Oxyhexyloxy)-1,2,5-thiadiazol-4-yl)-1,2,5,6-tetrahydro-1-methylpyridine;
3-(3-(3-Methyl-4-pentenyloxy)-1,2,5-thiadiazol-4-yl)-1,2,5,6-tetrahydro-1-methylpyridine;
3-(3-(4-Methylenecyclohexylmethyl)-1,2,5-thiadiazol-4-yl)-1,2,5,6-tetrahydro-1-methylpyridine;
3-(3-(2,3-Dimethylpentyloxy)-1,2,5-thiadiazol-4-yl)-1,2,5,6-tetrahydro-1-methylpyridine;
3-(3-(3-Cyclohexenylmethoxy)-1,2,5-thiadiazol-4-yl)-1,2,5,6-tetrahydro-1-methylpyridine;
3-(3-lsobutylthioethoxy-1,2,5-thiadiazol-4-yl)-1,2,5,6-tetrahydro-1-methylpyridine;
3-(3-Cyclopropylpropoxy-1,2,5-thiadiazol-4-yl)-1,2,5,6-tetrahydro-1-methylpyridine;
3-(3-(2-Methylcyclopropylmethoxy)-1,2,5-thiadiazol-4-yl)-1,2,5,6-tetrahydro-1-methylpyridine;
3-(3-Cyclopentylpropyloxy-1,2,5-thiadiazol-4-yl)-1,2,5,6-tetrahydro-1-methylpyridine;
3-(3-(4-Methylhexyloxy)-1,2,5-thiadiazol-4-yl)-1,2,5,6-tetrahydro-1-methylpyridine;
3-(3-(1-Methylhexyloxy)-1,2,5-thiadiazol-4-yl)-1,2,5,6-tetrahydro-1-methylpyridine;
3-(3-(4,4,4-Trifluorobutoxy)-1,2,5-thiadiazol-4-yl)-1,2,5,6-tetrahydro-1-methylpyridine;
3-(3-(3-Methylpentyloxy)-1,2,5-thiadiazol-4-yl)-1,2,5,6-tetrahydro-1-methylpyridine;
3-(3-(6,6,6-Trifluorohexyloxy)-1,2,5-thiadiazol-4 -y l)-1,2,5,6-tetrahydro-1-methylpyridine;
3-(3-(3-Cyclobutylpropoxy)-1,2,5-thiadiazol-4-yl)-1,2,5,6-tetrahydro-1-methylpyridine;
3-(3-Isopropoxyethoxy-1,2,5-thiadiazol-4-yl)-1,2,5,6-tetrahydro-1-methylpyridine;
3-(3-lsoheptyloxy-1,2,5-thiadiazol-4-yl)-1,2,5,6-tetrahydro-1-methylpyridine;
3-(3-Isohexyloxy-1,2,5-thiadiazol-4-yl)-1,2,5,6-tetrahydro-1-methylpyridine;
3-(3-(2,2,2-Trifluoroethoxy)-1,2,5-thiadiazol-4-yl)-1,2,5,6-tetrahydro-1-methylpyridine;
3-(3-(2-Chlorophenylpropoxy-1,2,5-thiadiazol-4-yl)-1,2,5,6-tetrahydro-1-methylpyridine;
3-(3-(3-Cyclohexylpropoxy)-1,2,5-thiadiazol-4-yl)-1,2,5,6-tetrahydro-1-methylpyridine;
3-(3-(2-Cyclohexylethoxy)-1,2,5-thiadiazol-4-yl)-1,2,5,6-tetrahydro-1-methylpyridine;
3-(3-Hexylthio-1,2,5-thiadiazol-4-yl)-1,2,5,6-tetrahydro-1-ethylpyridine;
3-(3-Ethylthio-1,2,5-thiadiazol-4-yl)-1,2,5,6-tetrahydro-1-ethylpyridine;
3-(3-Hexyloxy-1,2,5-thiadiazol-4-yl)-1,2,5,6-tetrahydre-1-ethylpyridine;
3-(3-Pentylthio-1,2,5-oxadiazol-4-yl)-1,2,5,6-tetrahydro-1-methylpyridine;
3-(3-Hexylthio-1,2,5-oxadiazol-4-yl)-1,2,5,6-tetrahydro-1-methylpyridine;
3-(3-(4-Pentynylthio)-1,2,5-oxadiazol-4-yl)-1,2,5,6-tetrahydro-1-methylpyridine;
3-(3-Ethoxy-1,2,5-thiadiazol-4-yl)-1,2,5,6-tetrahydropyridine;
3-(3-Ethylthio-1,2,5-thiadiazol-4-yl)-1,2,5,6-tetrahydropyridine;
3-(3-Propylthio-1,2,5-thiadiazol-4-yl)-1,2,5,6-tetrahydropyridine;
3-(3-Butylthio-1,2,5-thiadiazol-4-yl)-1,2,5,6-tetrahydropyridine;
3-(3-Pentylthio-1,2,5-thiadiazol-4-yl)-1,2,5,6-tetrahydropyridine;
3-(3-Hexylthio-1,2,5-thiadiazol-4-yl-1,2,5,6-tetrahydropyridine:
3-(3-(4-Pentynylthio)-1,2,5-thiadiazol-4-yl)-1,2,5,6-tetrahydropyridine;
3-(3-(2,2,2-Trifluoroethylthio)-1,2,5-thiadiazol-4-yl)-1,2,5,6-tetrahydropyridine;
3-(3-(2,2,2-Trifluoroethoxy)-1,2,5-thiadiazol-4-yl)-1,2,5,6-tetrahydropyridine;
3-(3-(2-Phenoxyethylthio)-1,2,5-thiadiazol-4-yl)-1,2,5,6-tetrahydropyridine;
3-(3-(2,2,2-Trifluoroethylthio)-1,2,5-thiadiazol-4-yl)-1,2,5,6-tetrahydro-1-methylpyridine;
3-(3-Isohexylthio-1,2,5-thiadiazol-4-yl)-1,2,5,6-tetrahydro-1-methylpyridine;
3-(3-Ethoxycarbonylpropylthio-1,2,5-thiadiazol-4-yl)-1,2,5,6-tetrahydro-1-methylpyridine;
3-(3-(2-(2-Thienylthio)ethylthio))-1,2,5-thiadiazol-4-yl)-1,2,5,6-tetrahydro-1-methylpyridine;
3-(3-(5-Ethyl-2-thienylmethylthio)-1,2,5-thiadiazol-4-yl)-1,2,5,6-tetrahydro-1-methylpyridine;
3-(3-(6-Hydroxyhexylthio)-1,2,5-thiadiazol-4-yl)-1,2,5,6-tetrahydro-1-methylpyridine;
3-(3-(3-Methyl-2-thienylmethylthio)-1,2,5-thiadiazol-4-yl)-1,2,5,6-tetrahydro-1-methylpyridine;
3-(3-(2-(2-Thienylthio)propylthio))-1,2,5-thiadiazol-4-yl)-1,2,5,6-tetrahydro-1-methylpyridine;
3-(3-(4-Ethoxy-1,2,5-thiadiazol-3-ylthio)-1,2,5-thiadiazol-4-yl)-1,2,5,6-tetrahydro-1-methylpyridine;
3-(3-(5-Methyl-2-thienylmethylthio)-1,2,5-thiadiazol-4-yl)-1,2,5,6-tetrahydro-1-methylpyridine;
3-(3-(4-Ethylthio-1,2,5-thiadiazol-3-ylthio)-1,2,5-thiadiazol-4-yl)-1,2,5,6-tetrahydro-1-methylpyridine;
3-(3-(4-Butylthio-1,2,5-thiadiazol-3-ylthio)-1,2,5-thiadiazol-4-yl)-1,2,5,6-tetrahydro-1-methylpyridine;
3-(3-(4-Propoxy-1,2,5-thiadiazol-3-ylthio)-1,2,5-thiadiazol-4-yl)-1,2,5,6-tetrahydro-1-methylpyridine;
cis 3-(3-(3-Hexenylthio)-1,2,5-thiadiazol-4-yl)-1,2,5,6-tetrahydro-1-methylpyridine;
3-(3-(1-Cyclopropylmethylthio)-1,2,5-thiadiazol-4-yl)-1,2,5,6-tetrahydro-1-methylpyridine;
3-(3-(1-Ethoxycarbonylpentylthio)-1,2,5-thiadiazol-4-yl)-1,2,5,6-tetrahydro-1-methylpyridine;
3-(3-(5-Hexenylthio)-1,2,5-thiadiazol-4-yl)-1,2,5,6-tetrahydro-1-methylpyridine;
3-(3-Cyclopentylthio-1,2,5-thiadiazol-4-yl)-1,2,5,6-tetrahydro-1-methylpyridine;
3-(3-(2-Methoxyethylthio)-1,2,5-thiadiazol-4-yl)-1,2,5,6-tetrahydro-1-methylpyridine;
3-(3-(2-(2-Ethoxymethoxy)-ethylthio)-1,2,5-thiadiazol-4-yl)-1,2,5,6-tetrahydro-1-methylpyridine;
3-(3-(4-Pentynylthio)-1,2,5-thiadiazol-4-yl)-1,2,5,6-tetrahydro-1-methylpyridine;
3-(3-Heptylthio-1,2,5-thiadiazol-4-yl)-1,2,5,6-tetrahydro-1-methylpyridine;
3-(3-(2-Ethylbutylthio)-1,2,5-thiadiazol-4-yl)-1,2,5,6-tetrahydro-1-methylpyridine;
3-(3-Cyclohexylmethylthio-1,2,5-thiadiazol-4-yl)-1,2,5,6-tetrahydro-1-methylpyridine;
3-(3-(7-Octenylthio)-1,2,5-thiadiazol-4-yl)-1,2,5,6-tetrahydro-1-methylpyridine;
3-(3-(3-Butenylthio)-1,2,5-thiadiazol-4-yl)-1,2,5,6-tetrahydro-1-methylpyridine;
3-(3-(4-Pentenylthio)-1,2,5- thiadiazol-4-yl)-1,2,5,6-tetrahydro-1-methylpyridine;
3-(3-(3,3,3-Trifluoropropylthio)-1,2,5-thiadiazol-4-yl)-1,2,5,6-tetrahydro-1-methylpyridine;
3-(3-(1-Oxo-1-phenylpropylthio-1,2,5-thiadiazol-4-yl)-1,2,5,6-tetrahydro-1-methylpyridine;
3-(3-(4-Phenylthiobutylthio)-1,2,5-thiadiazol-4-yl)-1,2,5,6-tetrahydro-1-methylpyridine;
3-(3-Cyanomethylthio-1,2,5-thiadiazol-4-yl)-1,2,5,6-tetrahydro-1-methylpyridine;
3-(3-(6-Chlorohexylthio)-1,2,5-thiadiazol-4-yl)-1,2,5,6-tetrahydro-1-methylpyridine;
3-(3-(5-Chloropentylthio)-1,2,5-thiadiazol-4-yl)-1,2,5,6-tetrahydro-1-methylpyridine;
3-(3-(3-Carboxypropylthio)-1,2,5-thiadiazol-4-yl)-1,2,5,6-tetrahydro-1-methylpyridine;
3-(3-(3-Carboxypropoxy)-1,2,5-thiadiazol-4-yl)-1,2,5,6-tetrahydro-1-methylpyridine;
3-(3-(5-Carboxypentylthio)-1,2,5-thiadiazol-4-yl)-1,2,5,6-tetrahydro-1-methylpyridine;
3-(3-(5-Mercaptopentylthio)-1,2,5-thiadiazol-4-yl)-1,2,5,6-tetrahydro-1-methylpyridine;
3-(3-(6-Mercaptohexylthio)-1,2,5-thiadiazol-4-yl)-1,2,5,6-tetrahydro-1-methylpyridine;
3-(3-(4-Mercaptobutylthio)-1,2,5-thiadiazol-4-yl)-1,2,5,6-tetrahydro-1-methpyridine;
or a pharmaceutically acceptable salt thereof.

Particularly Preferred compounds for use in treating anxiety include:
3-(3-METHOXY-1,2,5-THIADIAZOL-4-YL)-1,2,5,6-TETRAHYDRO-1-METHYLPYRIDINE
3-(3-ETHOXY-1,2,5-THIADIAZOL-4-YL)-1,2,5,6-TETRAHYDRO-1-METHYLPYRIDINE
3-(3-PROPOXY-1,2,5-THIADIAZOL-4-YL)-1,2,5,6-TETRAHYDRO-1-METHYLPYRIDINE
3-(3-BUTOXY-1,2,5-THIADIAZOL-4-YL)1,2,5,6-TETRAHYDRO-1-METHYLPYRIDINE
3-(3-ISOPROPOXY-1,2,5-THIADIAZOL-4-YL)-1,2,5,6-TETRAHYDRO-1-METHYLPYRIDINE
3-(3-CYCLOPROPYLMETHOXY-1,2,5-THIADIAZOL-4-YL)-1,2,5,6-TETRAHYDRO-1-METHYLPYRIDINE
3-(3-PENTOXY-1,2,5-THIADIAZOL-4-YL)-1,2,5,6-TETRAHYDRO-1-METHYLPYRIDINE
3-(3-ISOBUTOXY-1,2,5-THIADIAZOL-4-YL)-1,2,5,6-TETRAHYDRO-1-METHYLPYRIDINE
3-(3-(3-BUTENOXY)-1,2,5-THIADIAZOL-4-YL)-1,2,5,6-TETRAHYDRO-1-METHYLPYRIDINE
3-(3-(BUT-2-YNOXY)-1,2,5-THIADIAZOL-4-YL)-1,2,5,6-TETRAHYDRO-1-METHYLPYRIDINE
3-(3-(3-METHYLBUTOXY)-1,2,5-THIADIAZOL-4-YL)-1,2,5,6-TETRAHYDRO-1-METHYLPYRIDINE
3-(3-HEXYLOXY-1,2,5-THIADIAZOL-4-YL)-1,2,5,6-TETRAHYDRO-1-METHYLPYRIDINE
3-(3-(PROP-2-YNOXY)-1,2,5-THIADIAZOL-4-YL)-1,2,5,6-TETRAHYDRO-1-METHYLPYRIDINE
3-(3-BENZYLOXY-1,2,5-THIADIAZOL-4-YL)-1,2,5,6-TETRAHYDRO-1-METHYLPYRIDINE
3-(3-CHLORO-1,2,5-THIADIAZOL-4-YL)-1,2,5,6-TETRAHYDRO-1-METHYLPYRIDINE
3-(3-CHLORO-1,2,5-THIADIAZOL-4-YL)-1,2,5,6-TETRAHYDROPYRIDINE
3-(3-BUTOXY-1,2,5-THIADIAZOL-4-YL)-1,2,5,6-TETRAHYDROPYRIDINE
3-(3-ETHOXY-1,2,5-THIADIAZOL-4-YL)-1,2,5,6-TETRAHYDRO-1-ETHYLPYRIDINE
3-(3-CHLORO-1,2,5-THIADIAZOL-4-YL)-1,2,5,6-TETRAHYDRO-1-ETHYLPYRIDINE
3-(3-METHOXYETHOXY-1,2,5-THIADIAZOL-4-YL)-1,2,5,6-TETRAHYDRO-1-METHYLPYRIDINE
3-(3-HEPTYLOXY-1,2,5-THIADIAZOL-4-YL)-1,2,5,6-TETRAHYDRO-1-METHYLPYRIDINE
3-(3-(3-PENTYNYLOXY)-1,2,5-THIADIAZOL-4-YL)-1,2,5,6-TETRAHYDRO-1-METHYLPYRIDINE
3-(3-(4-PENTENYLOXY)-1,2,5-THIADIAZOL-4-YL)-1,2,5,6-TETRAHYDRO-1-METHYLPYRIDINE
3-(3-(2-PROPENYLOXY)-1,2,5-THIADIAZOL-4-YL)-1,2,5,6-TETRAHYDRO-1-METHYLPYRIDINE
3-(3-OCTYLOXY-1,2,5-THIADIAZOL-4-YL)-1,2,5,6-TETRAHYDRO-1-METHYLPYRIDINE
3-(3-(3-HEXYNYLOXY)-1,2,5-THIADIAZOL-4-YL)-1,2,5,6-TETRAHYDRO-1-METHYLPYRIDINE
3-(3-(3-BUTENYL-2-OXY)-1,2,5-THIADIAZOL-4-YL)-1,2,5,6-TETRAHYDRO-1-METHYL-PYRIDINE
3-(3-(4-HEXENYLOXY(-1,2,5-THIADIAZOL-4-YL)-1,2,5,6-TETRAHYDRO-1-METHYLPYRIDINE
TRANS-3-(3-(3-HEXENYLOXY)-1,2,5-THIADIAZOL-4-YL)-1,2,5,6-TETRAHYDRO-1-METHYLPYRIDINE
CIS-3-(3-(2-PENTENYLOXY)-1,2,5-THIADIAZOL-4-YL)-1,2,5,6-TETRAHYDRO-1-METHYLPYRIDINE
CIS-3-(3-(2-HEXENYLOXY)-1,2,5-THIADIAZOL-4-YL)-1,2,5,6-TETRAHYDRO-1-METHYLPYRIDINE
3-(3-(5-HEXENYLOXY)-1,2,5-THIADIAZOL-4-YL)-1,2,5,6-TETRAHYDRO-1-METHYLPYRIDINE
CIS-3-(3-(3-HEXENYLOXY)-1,2,5-THIADIAZOL-4-YL)-1,2,4,5-TETRAHYDRO-1-METHYLPYRIDINE
TRANS-3-(3-(2-HEXENYLOXY)-1,2,5-THIADIAZOL-4-YL)-1,2,5,6-TETRAHYDRO-1-METHYLPYRIDINE
3-(1,2,5-THIADIAZOL-4-YL)-1,2,5,6-TETRAHYDRO-1-METHYLPYRIDINE
3-(3-(4-METHYLPIPERIDINO-1,2,5-THIADIAZOL-4-YL)-1,2,5,6-TETRAHYDRO-1-METHYLPYRIDINE
3-(3-MORPHOLINO-1,2,5-THIADIAZOL-4-YL)-1,2,5,6-TETRAHYDRO-1-METHYLPYRIDINE
3-(3-DIMETHYLAMINO-1,2,5-THIADIAZOL-4-YL)-1,2,5,6-TETRAHYDRO-1-METHYLPYRIDINE
3-(3-HEXYLAMINO-1,2,5-THIADIAZOL-4-YL)-1,2,5,6-TETRAHYDRO-1-METHYLPYRIDINE
3-(3-HEXYLOXY-1,2,5-THIADIAZOL-4-YL)-1,2,5,6-TETRAHYDRO-1-DEUTEROMETHYLPYRIDINE
1,2,5,6-TETRAHYDRO-3-(3-HEXYLOXY-1,2,5-THIADIAZOL-4-YL)PYRIDINE
3-(3-(2-(2-METHOXYETHOXY)-ETHOXY)-1,2,5-THIADIAZOL-4-YL)-1,2,5,6-TETRAHYDRO-1-METHYLPYRIDINE
3-(3-(3-ETHOXY-1-PROPOXY)-1,2,5-THIADIAZOL-4-YL)-1,2,5,6,-TETRAHYDRO-1-METHYLPYRIDINE
3-(3-(2-ETHOXYETHOXY)-1,2,5-THIADIAZOL-4-YL)-1,2,5,6-TETRAHYDRO-1-METHYLPYRIDINE
3-(3-(2-BUTOXYETHOXY)-1,2,5-THIADIAZOL-4-YL)-1,2,5,6-TETRAHYDRO-1-METHYLPYRIDINE
3-(3-(2-(2-BUTOXYETHOXY)-ETHOXY)-1,2,5-THIADIAZOL-4-YL)-1,2,5,6-TETRAHYDRO-1-METHYLPYRIDINE
3-(3-(2-(2-ETHOXYETHOXY)-ETHOXY)-1,2,5-THIADIAZOL-4-YL)-1,2,5,6-TETRAHYDRO-1-METHYLPYRIDINE
3-(3-BUTYLTHIO-1,2,5-THIADIAZOL-4-YL)-1,2,5,6-TETRAHYDRO-1-METHYLPYRIDINE
3-(3-METHYLTHIO-1,2,5-THIADIAZOL-4-YL)-1,2,5,6-TETRAHYDRO-1-METHYLPYRIDINE
3-(3-PENTYL-1,2,5-THIADIAZOL-4-YL)-1,2,5,6-TETRAHYDRO-1-METHYLPYRIDINE
3-(3-PROPYLTHIO-1,2,5-THIADIAZOL-4-YL)-1,2,5,6-TETRAHYDRO-1-METHYLPYRIDINE
3-(3-HEXYLTHIO-1,2,5-THIADIAZOL-4-YL)-1,2,5,6-TETRAHYDRO-1-METHYLPYRIDINE
3-(3-PENTYLTHIO-1,2,5-THIADIAZOL-4-YL)-1,2,5,6-TETRAHYDRO-1-METHYLPYRIDINE
3-(3-ETHYLTHIO-1,2,5-THIADIAZOL-4-YL)-1,2,5,6-TETRAHYDRO-1-METHYLPYRIDINE
3-(3-OCTYLTHIO-1,2,5-THIADIAZOL-4-YL)-1,2,5,6-TETRAHYDRO-1-METHYLPYRIDINE
3-(3-PROPYL-1,2,5-THIADIAZOL-4-YL)-1,2,5,6-TETRAHYDRO-1-METHYLPYRIDINE
3-(3-HEPTYL-1,2,5-THIADIAZOL-4-YL)-1,2,5,6-TETRAHYDRO-1-METHYLPYRIDINE
3-(3-(5-HEXENYL)-1,2,5-THIADIAZOL-4-YL)-1,2,5,6-TETRAHYDRO-1-METHYLPYRIDINE
3-(3-OCTYL-1,2,5-THIADIAZOL-4-YL)-1,2,5,6-TETRAHYDRO-1-METHYPYRIDINE
3-(3-(2-METHYL)-BUTYL-1,2,5-THIADIAZOL-4-YL)-1,2,5,6-TETRAHYDRO-1-METHYLPYRIDINE
3-(3-METHYLCYCLOPROPYL-1,2,5-THIADIAZOL-4-YL)-1,2,5,6-TETRAHYDRO-1-METHYLPYRIDINE
3-(3-CYCLOPENTYLTHIO-1,2,5-THIADIAZOL-4-YL)-1,2,5,6-TETRAHYDRO-1-METHYLPYRIDINE
3-(3-(1-ETHYLTHIO-2-METHOXY)-1,2,5-THIADIAZOL-4-YL)-1,2,5,6-TETRAHYDRO-1-METHYLPYRIDINE
3-(3-(3-CHLORO-1-PROPYLTHIO)-1,2,5-THIADIAZOL-4-YL)-1,2,5,6-TETRAHYDRO-1-METHYLPYRIDINE
3-(3-(2-METHOXYETHOXY)-ETHYLTHIO)-1,2,5-THIADIAZOL-4-YL)-1,2,5,6-TETRAHYDRO-1-METHYLPYRIDINE
3-(3-(3-CYANO-1-PROPYLTHIO)-1,2,5-THIADIAZOL-4-YL)-1,2,5,6-TETRAHYDRO-1-METHYLPYRIDINE
3-(3-BENZYLTHIO-1,2,5-THIADIAZOL-4-YL)-1,2,5,6-TETRAHYDRO-1-METHYLPYRIDINE
3-(3-(2-ETHOXY-1-ETHYLTHIO)-1,2,5-THIADIAZOL-4-YL)-1,2,5,6-TETRAHYDRO-1-METHYLPYRIDINE
3-(3-(4-PENTYNYLTHIO)-1,2,5-THIADIAZOL-4-YL)-1,2,5,6-TETRAHYDRO-1-METHYLPYRIDINE
3-(3-(2-(2-ETHOXYMETHOXY)-ETHYLTHIO)-1,2,5-THIADIAZOL-4-YL)-1,2,5,6-TETRAHYDRO-1-METHYLPYRIDINE
3-(3-(5-CYANO-1-PENTYLTHIO)-1,2,5-THIADIAZOL-4-YL)-1,2,5,6-TETRAHYDRO-1-METHYLPYRIDINE
3-(3-(3-PHENYL-1-PROPYLTHIO)-1,2,5-THIADIAZOL-4-YL)-1,2,5,6-TETRAHYDRO-1-METHYLPYRIDINE
3-(3-(2-PHENOXYETHYLTHIO)-1,2,5-THIADIAZOL-4-YL)-1,2,5,6-TETRAHYDRO-1-METHYLPYRIDINE
3-(3-(4-CYANOBUTYLTHIO)-1,2,5-THIADIAZOL-4-YL)-1,2,5,6-TETRAHYDRO-1-METHYLPYRIDINE
3-(3-(2-ETHYLBUTYLTHIO)-1,2,5-THIADIAZOL-4-YL)-1,2,5,6-TETRAHYDRO-1-METHYLPYRIDINE
3-(3-CYCLOHEXYLMETHYLTHIO-1,2,5-THIADIAZOL-4-YL)-1,2,5,6-TETRAHYDRO-1-METHYLPYRIDINE
3-(3-(8-HYDROXYOCTYLTHIO)-1,2,5-THIADIAZOL-4-YL)-1,2,5,6-TETRAHYDRO-1-METHYLPYRIDINE
3-(3-(7-OCTENYLTHIO)-1,2,5-THIADIAZOL-4-YL)-1,2,5,6-TETRAHYDRO-1-METHYLPYRIDINE
3-(3-CYCLOPROPYLMETHYLTHIO-1,2,5-THIADIAZOL-4-YL)-1,2,5,6-TETRAHYDRO-1-METHYLPYRIDINE
3-(3-(3-BUTENYLTHIO)-1,2,5-THIADIAZOL-4-YL)-1,2,5,6-TETRAHYDRO-1-METHYLPYRIDINE
3-(3-(4-PENTENYLTHIO)-1,2,5-THIADIAZOL-4-YL)-1,2,5,6-TETRAHYDRO-1-METHYLPYRIDINE
3-(4-ISOHEXYLOXY-1,2,5-THIADIAZOL-3-YL)-1,2,5,6-TETRAHYDRO-1-METHYLPYRIDINE
1-METHYL-1,2,5,6-TETRAHYDRO-3-((4-CYCLOPENTYLPROPYL)OXY-1,2,5-THIADIAZOL-3-YL)PYRIDINE
1-METHYL-1,2,5,6-TETRAHYDRO-3-(4-ISOHEPTYLOXY-1,2,5-THIADIAZOL-3-YL)PYRIDINE
1-METHYL-1,2,5,6-TETRAHYDRO-3-(4((2-CYCLOHEXYLETHYL)OXY)-1,2,5-THIADIAZOL-3-YL)PYRIDINE
1,2,5,6-TETRAHYDRO-1-METHYL-3-(4-(1-METHYLHEXYLOXY)-1,2,5-THIADIAZOL-3-YL)PYRIDINE
3-(4-(1-ETHYLPENTYLOXY)-1,2,5-THIADIAZOL-3-YL)-1,2,5,6-TETRAHYDRO-1-METHYLPYRIDINE
3-(4-(1-ETHYLBUTOXY)-1,2,5-THIADIAZOL-3-YL)-1,2,5,6-TETRAHYDRO-1-METHYLPYRIDINE
1,2,5,6-TETRAHYDRO-1-METHYL-3-(4-(1-METHYLPENTYLOXY)-1,2,5-THIADIAZOL-3-YL)PYRIDINE
1-METHYL-3-(4-(5-HEXENYLOXY-1,2,5-THIADIAZOL-3-YL)-1,2,5,6-TETRAHYDROPYRIDINE
1,2,5,6-TETRAHYDRO-1-METHYL-3-(4-(2-METHYLBUTOXY)-1,2,5-THIADIAZOL-3-YL)PYRIDINE
1,2,5,6-TETRAHYDRO-1-METHYL-3-(4-(2-METHYLPENTYLOXY)-1,2,5-THIADIAZOL-3-YL)PYRIDINE
1,2,5,6-TETRAHYDRO-1-METHYL-3-(4-(2,2,2-TRIFLUOROETHOXY)-1,2,5-THIADIAZOL-3-YL)PYRIDINE
1-METHYL-1,2,5,6-TETRAHYDRO-3-(4-(3-METHYLPENTYLOXY)-1,2,5-THIADIAZOL-3-YL)PYRIDINE
3-(3-(3-METHYL-2-BUTENYLOXY)-1,2,5-THIADIAZOL-4-YL)-1,2,5,6-TETRAHYDO-1-METHYLPYRIDINE
3-(3-ISOBUTOXY-1,2,5-THIADIAZOL-4-YL)-1,2,5,6-TETRAHYDRO-1-METHYLPYRIDINE
1,2,5,6-TETRAHYDRO-1-METHYL-3-(4-(2-METHYLBUTOXY)-1,2,5-THIADIAZOL-3-YL)PYRIDINE
3-(3-(3-HYDROXYPROPOXY)-1,2,5-THIADIAZOL-4-YL)-1,2,5,6-TETRAHYDRO-1-METHYLPYRIDINE
(+-)1,6-DIMETHYL-3-(3-HEXYLOXY-1,2,5-THIADIAZOL-4-YL)-1,2,5,6-TETRAHYDROPYRIDINE
3-(3-(3-PHENYL-ETHYLTHIO)-1,2,5-THIADIAZOL-4-YL)-1,2,5,6-TETRAHYDRO-1-METHYLPYRIDINE
BIS-1,4-(3-(1-METHYL-1,2,5,6-TETRAHYDROPYRIDIN-3-YL)-1,2,5-THIADIAZOL-4-YL)BUTANEDITHIOL
3-(3-(4,4,4-TRIFLUOROBUTOXY)-1,2,5-THIADIAZOL-4-YL)-1,2,5,6-TETRAHYDRO-1-METHYLPYRIDINE
3-(3-(3,3,3-TRIFLUOROPROPYLTHIO)-1,2,5-THIADIAZOL-4-YL)-1,2,5,6-TETRAHYDRO-1-METHYLPYRIDINE
3-(3-PROPYLTHIO-1,2,5-THIADIAZOL-4-YL)-1,2,5,6-TETRAHYDROPYRIDINE
3-(3-BUTYLTHIO-1,2,5-THIADIAZOL-4-YL)-1,2,5,6-TETRAHYDROPYRIDINE
3-(3-BUTYLTHIO-1,2,5-THIADIAZOL-4-YL)-1,2,5,6-TETPAHYDRO-1,1-DIMETHYLPYRIDINIUM IODIDE
(+-)1,6-DIMETHYL-3-(3-BUTYLTHIO-1,2,5-THIADIAZOL-4-YL)-1,2,5,6-TETRAHYDROPYRIDINE
(+-)1,6-DIMETHYL-3-(3-BUTOXY-1,2,5-THIADIAZOL-4-YL)-1,2,5,6-TETRAHYDROPYRIDINE; or
a pharmaceutically acceptable salt thereof.

Especially preferred compounds include the following:
3-(3-BUTYLTHIO-1,2,5-THIADIAZOL-4-YL)-1,2,5,6-TETRAHYDRO-1-METHYLPYRIDINE
3-(3-METHYLTHIO-1,2,5-THIADIAZOL-4-YL)-1,2,5,6-TETRAHYDRO-1-METHYLPYRIDINE
3-(3-PENTYL-1,2,5-THIADIAZOL-4-YL)-1,2,5,6-TETRAHYDRO-1-METHYLPYRIDINE
3-(3-PROPYLTHIO-1,2,5-THIADIAZOL-4-YL)-1,2,5,6-TETRAHYDRO-1-METHYLPYRIDINE
3-(3-HEXYLTHIO-1,2,5-THIADIAZOL-4-YL)-1,2,5,6-TETRAHYDRO-1-METHYLPYRIDINE
3-(3-PENTYLTHIO-1,2,5-THIADIAZOL-4-YL)-1,2,5,6-TETRAHYDRO-1-METHYLPYRIDINE
3-(3-ETHYLTHIO-1,2,5-THIADIAZOL-4-YL)-1,2,5,6-TETRAHYDRO-1-METHYLPYRIDINE
3-(3-OCTYLTHIO-1,2,5-THIADIAZOL-4-YL)-1,2,5,6-TETRAHYDRO-1-METHYLPYRIDINE
3-(3-METHOXY-1,2,5-THIADIAZOL-4-YL)-1,2,5,6-TETRAHYDRO-1-METHYLPYRIDINE
3-(3-ETHOXY-1,2,5-THIADIAZOL-4-YL)-1,2,5,6-TETRAHYDRO-1-METHYLPYRIDINE
3-(3-PROPOXY-1,2,5-THIADIAZOL-4-YL)-1,2,5,6-TETRAHYDRO-1-METHYLPYRIDINE
3-(3-BUTOXY-1,2,5-THIADIAZOL-4-YL)1,2,5,6-TETTRAHYDRO-1-METHYLPYRIDINE
3-(3-ISOPROPOXY-1,2,5-THIADIAZOL-4-YL)-1,2,5,6-TETRAHYDRO-1-METHYLPYRIDINE
3-(3-CYCLOPROPYLMETHOXY-1,2,5-THIADIAZOL-4-YL)-1,2,5,6-TETRAHYDRO-1-METHYLPYRIDINE
3-(3-PENTOXY-1,2,5-THIADIAZOL-4-YL)-1,2,5,6-TETRAHYDRO-1-METHYLPYRIDINE
3-(3-ISOBUTOXY-1,2,5-THIADIAZOL-4-YL)-1,2,5,6-TETRAHYDRO-1-METHYLPYRIDINE
3-(3-(3-BUTENOXY)-1,2,5-THIADIAZOL-4-YL)-1,2,5,6-TETRAHYDRO-1-METHYLPYRIDINE
3-(3-(BUT-2-YNOXY)-1,2,5-THIADIAZOL-4-YL)-1,2,5,6-TETRAHYDRO-1-METHYLPYRIDINE
3-(3-(3-METHYLBUTOXY)-1,2,5-THIADIAZOL-4-YL)-1,2,5,6-TETRAHYDRO-1-METHYLPYRIDINE
3-(3-HEXYLOXY-1,2,5-THIADIAZOL-4-YL)-1,2,5,6-TETRAHYDRO-1-METHYLPYRIDINE
3-(3-(PROP-2-YNOXY)-1,2,5-THIADIAZOL-4-YL)-1,2,5,6-TETRAHYDRO-1-METHYLPYRIDINE; or
a pharmaceutically acceptable salt thereof.

Compound which are particularly preferred include:
3-(3-HEXYLOXY-1,2,5-THIADIAZOL-4-YL)-1,2,5,6-TETRAHYDRO-1-METHYLPYRIDINE;
3-(3-HEXYLTHIO-1,2,5-THIADIAZOL-4-YL)-1,2,5,6-TETRAHYDRO-1-METHYLPYRIDINE; or
a pharmaceutically acceptable salt thereof.

## Claims

1. Use of a compound of Formula I: 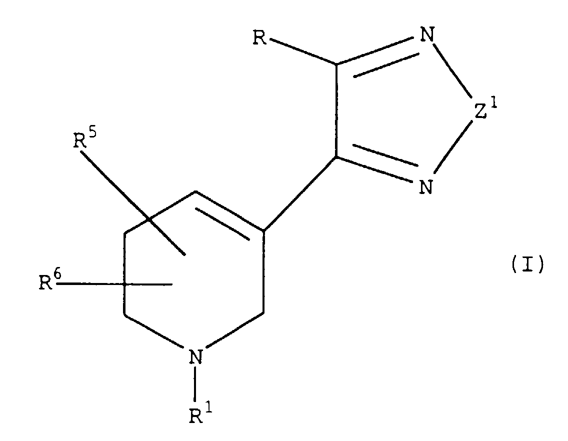 wherein
Z1 is oxygen or sulphur;
R is hydrogen, halogen, amino, -NHCO-R², C₃₋₇-cycloalkyl, C₄₋₁₀-(cycloalkylalkyl), -Z²-C₃₋₇-cycloalkyl optionally substituted with C₁₋₆-alkyl, -Z²-C₄₋₁₀-(cycloalkylalkyl), -Z²⁻C₄₋₁₀-(cycloalkenylalkyl), -Z²-C₄₋₁₀-(methylenecycloalkyl-alkyl), -NH-R2, -NR2R3, -NH-OR2, phenyl, phenoxy, benzoyl, benzyloxycarbonyl, tetrahydronaphtyl, indenyl, X, R², -Z²R², -SOR², -SO₂R², -Z²-R²-Z³-R³, -Z²-R²-Z³-R³-Z⁴-R⁴, -Z²-R²CO-R³, -Z²-R²-CO₂-R³, Z²-R²-O₂C-R³, -Z²-R²-CONH-R³, -2²⁻R²-NHCOR³, -Z²-R²-X, -Z²-R²-Z³-X, wherein Z², Z³, and Z⁴ independently are oxygen or sulphur, and R², R³ and R⁴ independently are straight or branched C₁₋₁₅-alkyl, straight or branched C₂₋₁₅-alkenyl, straight or branched C₂₋₁₅-alkynyl, each of which is optionally substituted with halogen(s), -OH, -CN, -CF₃, -SH, -COOH, -NH-R², -NR²R³, C₁₋₆alkyl ester, one or two phenyl, phenoxy, benzoyl or benzyloxycarbonyl wherein each aromatic group is optionally substituted with one or two halogen, -CN, C₁₋₄-alkyl or C₁₋₄-alkoxy, and X is a 5 or 6 membered heterocyclic group containing one to four N, 0 or S atom(s) or a combination thereof, which heterocyclic group is optionally substituted at carbon or nitrogen atom(s) with straight or branched C₁₋₆-alkyl, phenyl, benzyl or pyridine, or a carbon atom in the heterocyclic group together with an oxygen atom form a carbonyl group, or which heterocyclic group is optionally fused with a phenyl group; and
R⁵ and R⁶ may be present at any position, including the point of attachment of the thiadiazole or oxadiazole ring, and independently are hydrogen, straight or branched C₁₋₅-alkyl, straight or branched C₂₋₅-alkenyl, straight or branched C₂₋₅-alkynyl, straight or branched C₁₋₁₀-alkoxy, straight or branched C₁₋₅-alkyl substituted with -OH, halogen, -NH₂ or carboxy; R¹ is hydrogen, straight or branched C₁₋₅-alkyl, straight or branched C₂₋₅-alkenyl or straight or branched C₂₋₅-alkynyl; or
a pharmaceutically acceptable salt or solvate thereof; for the manufacture of a medicament for the treatment of anxiety.

2. The use of a compound of Formula I according to Claim 1 wherein Z¹ is sulphur; or a pharmaceutically acceptable salt thereof.

3. The use of a compound of Formula I 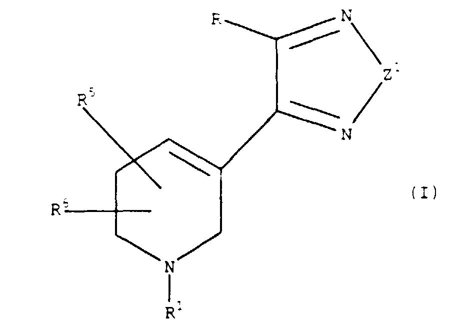 wherein R is as defined in Claim 1, and Z¹ is sulphur, R¹ is hydrogen or straight or branched C₁₋₅-alkyl, R⁵ and R⁶ independently are hydrogen, methyl, methoxy, hydroxy, halogen or amino; or a pharmaceutically acceptable salt thereof; for the manufacture of a medicament for the treatment of anxiety.

4. The use of a compound of Formula I according to Claim 1 wherein Z¹ is sulphur, R¹ is hydrogen or methyl, R⁵ and R⁶ are hydrogen, R is -Z²R² wherein Z² is oxygen or sulphur and R² is straight or branched C₁₋₁₅-alkyl; or a pharmaceutically acceptable salt thereof.

5. The use of a compound of Formula I according to Claim 1 wherein Z¹ is sulphur, R¹ is hydrogen or methyl, R⁵ and R⁶ are hydrogen, R is -Z²R² wherein Z² is oxygen or sulphur and R² is straight or branched C₁₋₁₅-alkyl substituted with halogen(s) or -CF₃; or a pharmaceutically acceptable salt thereof.

## Patentansprüche

1. Verwendung einer Verbindung der Formel I 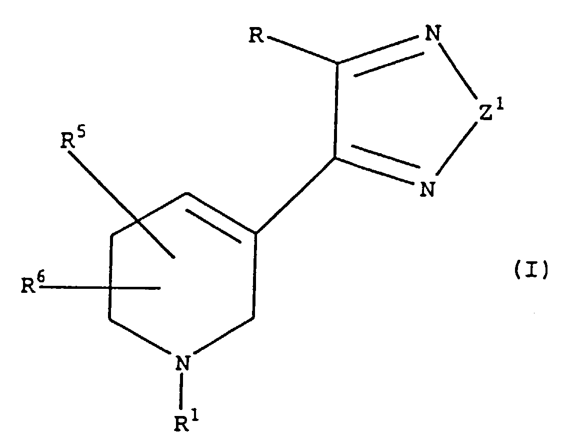 worin Z¹ für Sauerstoff oder Schwefel steht,
R für Wasserstoff, Halogen, Amino, -NHCO-R², C₃-C₇ Cycloalkyl, C₄-C₁₀ Cycloalkylalkyl, wahlweise mit C₁-C₆ Alkyl substituiertes -Z²-C₃-C₇ Cycloalkyl, -Z²-C₄-C₁₀ Cycloalkylalkyl, -Z²-C₄-C₁₀ Cycloalkenylalkyl, -Z²-C₄-C₁₀ Methylencycloalkylalkyl, -NH-R², -NR²R³, -NH-OR², Phenyl, Phenoxy, Benzoyl, Benzyloxycarbonyl, Tetrahydronaphthyl, Indenyl, X, R², -Z²R², -SOR², -SO₂R², -Z²-R²-Z³-R³, -Z²-R²-Z³-R³-Z⁴-R⁴, -Z²-R²-CO-R³, -Z²-R²-CO₂-R³, -Z²-R²-O₂C-R³, -Z²-R²-CONH-R³, -Z²-R²-NHCOR³, -Z²-R²-X, -Z²-R²-Z³-X, worin Z², Z³ und Z⁴ unabhängig für Sauerstoff oder Schwefel stehen und R², R³ und R⁴ unabhängig für gerades oder verzweigtes C₁-C₁₅ Alkyl, gerades oder verzweigtes C₂-C₁₅ Alkenyl, gerades oder verzweigtes C₂-C₁₅ Alkinyl stehen, wobei jedes wahlweise substituiert ist mit Halogen(en), -OH, -CN, -CF₃, -SH, -COOH, -NH-R², -NR²R³, C₁-C₆ Alkylester, einem oder zwei Phenyl, Phenoxy, Benzoyl oder Benzyloxycarbonyl, worin jede aromatische Gruppe wahlweise substituiert ist mit ein oder zwei Halogenen, -CN, C₁-C₄ Alkyl oder C₁-C₄ Alkoxy und X für eine fünf- oder sechsgliedrige heterocyclische Gruppe, die ein bis vier N-, O- oder S-Atome enthält oder eine Kombination hiervon steht, wobei die heterocyclische Gruppe wahlweise an den Kohlenstoff- oder Stickstoffatomen mit geradem oder verzweigtem C₁-C₆ Alkyl, Phenyl, Benzyl oder Pyridin substituiert ist, oder ein Kohlenstoffatom in der heterocyclischen Gruppe zusammen mit einem Sauerstoffatom eine Carbonylgruppe bildet, oder worin die heterocyclische Gruppe wahlweise mit einer Phenylgruppe fusioniert ist, und
R⁵ und R⁶ an jeder Position stehen können, einschließlich der Bindungsstelle des Thiadiazol- oder Oxadiazolrings, und unabhängig stehen für Wasserstoff, gerades oder verzweigtes C₁-C₅ Alkyl, gerades oder verzweigtes C₂-C₅ Alkenyl, gerades oder verzweigtes C₂-C₅ Alkinyl, gerades oder verzweigtes C₁-C₁₀ Alkoxy, gerades oder verzweigtes C₁-C₅ Alkyl, das substituiert ist mit -OH, Halogen, -NH₂ oder Carboxy,
R₁ für Wasserstoff, gerades oder verzweigtes C₁-C₅ Alkyl, gerades oder verzweigtes C₂-C₅ Alkenyl oder gerades oder verzweigtes C₂-C₅ Alkinyl steht, oder
eines pharmazeutisch annehmbaren Salzes oder Solvats hiervon zur Herstellung eines Arzneimittels zur Behandlung eines Angstzustands.

2. Verwendung einer Verbindung der Formel I nach Anspruch 1, worin Z¹ für Schwefel steht, oder eines pharmazeutisch annehmbaren Salzes hiervon.

3. Verwendung einer Verbindung der Formel I 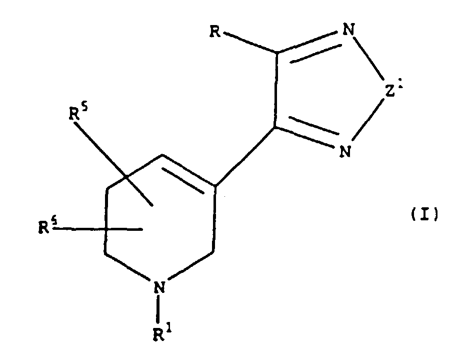 worin R wie in Anspruch 1 definiert ist und Z¹ für Schwefel steht, R¹ für Wasserstoff oder gerades oder verzweigtes C₁-C₅ Alkyl steht, R₅ und R₆ unabhängig für Wasserstoff, Methyl, Methoxy, Hydroxy, Halogen oder Amino stehen oder eines pharmazeutisch annehmbaren Salzes hiervon zur Herstellung eines Arzneimittels zur Behandlung eines Angstzustands.

4. Verwendung einer Verbindung der Formel I nach Anspruch 1, worin Z¹ für Schwefel steht, R¹ für Wasserstoff oder Methyl steht, R⁵ und R⁶ für Wasserstoff stehen, R für -Z²R² steht, worin Z² für Sauerstoff oder Schwefel steht und R² für gerades oder verzweigtes C₁-C₁₅ Alkyl steht, oder eines pharmazeutisch annehmbaren Salzes hiervon.

5. Verwendung einer Verbindung der Formel I nach Anspruch 1, worin Z¹ für Schwefel steht, R¹ für Wasserstoff oder Methyl steht, R⁵ und R⁶ für Wasserstoff stehen, R für -Z²R² steht, worin Z² für Sauerstoff oder Schwefel steht und R² für gerades oder verzweigtes C₁-C₁₅ Alkyl steht, das mit Halogen(en) oder -CF₃ substituiert ist, oder eines pharmazeutisch annehmbaren Salzes hiervon.

## Revendications

1. Utilisation d'un composé répondant à la formule I: 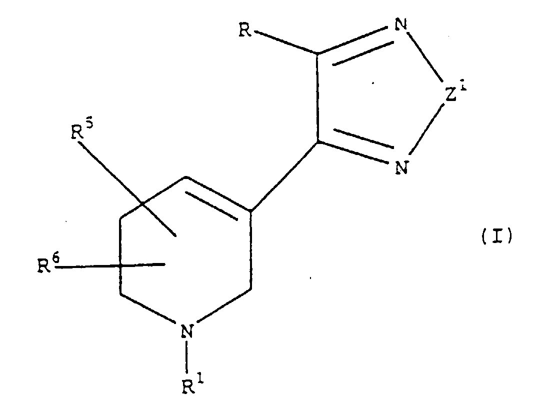 dans laquelle
Z¹ représente un atome d'oxygène ou un atome de soufre;
R représente un atome d'hydrogène, un atome d'halogène, un groupe amino, un groupe -NHCO-R², un groupe cycloalkyle en C₃-C₇, un groupe cycloalkylalkyle en C₄-C₁₀, un groupe -Z²-cycloalkyle en C₃-C₇ portant le cas échéant un ou plusieurs substituants alkyle en C₁-C₆, un groupe -Z²-cycloalkylalkyle en C₄-C₁₀, un groupe -Z²-cycloalcénylalkyle en C₄-C₁₀, un groupe -Z²-méthylènecycloalkylalkyle en C₄-C₁₀, un groupe -NH-R², un groupe -NR²R³, un groupe -NH-OR², un groupe phényle, un groupe phénoxy, un groupe benzoyle, un groupe benzyloxycarbonyle, un groupe tétrahydronaphtyle, un groupe indényle, un groupe X, un groupe R², un groupe -Z²R², un groupe -SOR², un groupe -SO₂R², un groupe -Z²-R²-Z³-R³, un groupe -Z²-R²-Z³-R³-Z⁴-R⁴, un groupe -Z²-R²CO-R³, un groupe -Z²-R²-CO₂-R³, un groupe -Z²-R²-O₂C-R₃, un groupe -Z²-R²-CONH-R³, un groupe -Z²-R²-NHCOR³, un groupe -Z²-R²-X, un groupe -Z²-R²-Z³-X, dans lesquels Z², Z³ et Z⁴ représentent, indépendamment l'un de l'autre, un atome d'oxygène ou un atome de soufre, et R², R³ et R⁴ représentent, indépendamment l'un de l'autre, un groupe alkyle en C₁-C₁₅ à chaîne droite ou ramifiée, un groupe alcényle en C₂-C₁₅ à chaîne droite ou ramifiée, un groupe alcynyle en C₂-C₁₅ à chaîne droite ou ramifiée, chacun de ces groupes portant le cas échéant un ou plusieurs substituants identiques ou différents halogéno, -OH, -CN, -CF₃, -SH, -COOH, -NH-R², -NR²R³, ester alkylique en C₁-C₆; un ou deux substituants phényle, phénoxy, benzoyle ou benzyloxycarbonyle, chaque groupe aromatique portant le cas échéant un ou deux substituants identiques ou différents halogéno, -CN, alkyle en C₁-C₄ ou alcoxy en C₁-C₄, et X représente un groupe hétérocyclique à cinq ou six membres contenant de un à quatre atomes d'azote, d'oxygène ou de soufre ou une combinaison de ces derniers, ledit groupe hétérocyclique portant le cas échéant, sur l'atome ou sur les atomes de carbone ou d'azote, un ou plusieurs substituants alkyle en C₁-C₆ à chaînes droites ou ramifiées, phényle, benzyle ou pyridine, ou bien un atome de carbone dans le groupe hétérocyclique forme, conjointement avec un atome d'oxygène, un groupe carbonyle, ou bien ledit groupe hétérocyclique est le cas échéant condensé avec un groupe phényle; et
R⁵ et R⁶ peuvent être présents à n'importe quelle position, y compris à l'endroit de fixation du noyau thiadiazole ou du noyau oxadiazole et ils représentent, indépendamment l'un de l'autre, un atome d'hydrogène, un groupe alkyle en C₁-C₅ à chaîne droite ou ramifiée, un groupe alcényle en C₂-C₅ à chaîne droite ou ramifiée, un groupe alcynyle en C₂-C₅ à chaîne droite ou ramifiée, un groupe alcoxy en C₁-C₁₀ à chaîne droite ou ramifiée, un groupe alkyle en C₁-C₅ à chaîne droite ou ramifiée portant un ou plusieurs substituants identiques ou différents -OH, halogéno, -NH₂ ou carboxyle; R¹ représente un atome d'hydrogène, un groupe alkyle en C₁-C₅ à chaîne droite ou ramifiée, un groupe alcényle en C₂-C₅ à chaîne droite ou ramifiée ou un groupe alcynyle en C₂-C₅ à chaîne droite ou ramifiée;
ou d'un de ses sels ou de ses solvates pharmaceutiquement acceptables pour la formulation d'un médicament pour le traitement de l'anxiété.

2. Utilisation d'un composé répondant à la formule I selon la revendication 1, dans lequel Z¹ représente un atome de soufre, ou d'un de ses sels pharmaceutiquement acceptables.

3. Utilisation d'un composé répondant à la formule I 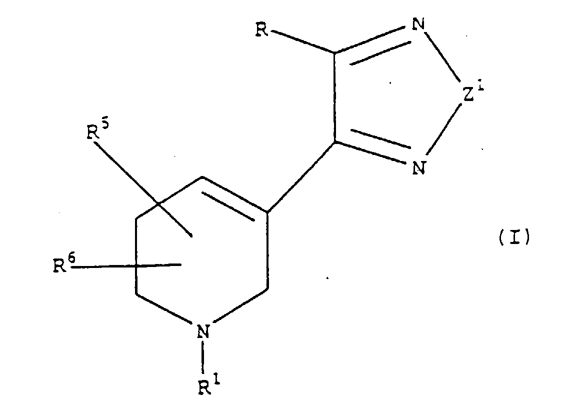 dans laquelle R est tel que défini à la revendication 1, et Z¹ représente un atome de soufre, R¹ représente un atome d'hydrogène ou un groupe alkyle en C₁-C₅ à chaîne droite ou ramifiée, R⁵ et R⁶ représentent, indépendamment l'un de l'autre, un atome d'hydrogène, un groupe méthyle, un groupe méthoxy, un groupe hydroxyle, un atome d'halogène ou un groupe amino, ou d'un de ses sels pharmaceutiquement acceptables pour la formulation d'un médicament pour le traitement de l'anxiété.

4. Utilisation d'un composé répondant à la formule I selon la revendication 1, dans lequel Z¹ représente un atome de soufre, R¹ représente un atome d'hydrogène ou un groupe méthyle, R⁵ et R⁶ représentent un atome d'hydrogène, R représente un groupe -Z²R² dans lequel Z² représente un atome d'oxygène ou un atome de soufre, et R² représente un groupe alkyle en C₁-C₁₅ à chaîne droite ou ramifiée, ou d'un de ses sels pharmaceutiquement acceptables.

5. Utilisation d'un composé répondant à la formule I selon la revendication 1, dans lequel Z¹ représente un atome de soufre, R¹ représente un atome d'hydrogène ou un groupe méthyle, R⁵ et R⁶ représentent un atome d'hydrogène, R représente un groupe -Z²R² dans lequel Z² représente un atome d'oxygène ou un atome de soufre, et R² représente un groupe alkyle en C₁-C₁₅ à chaîne droite ou ramifiée portant un ou plusieurs substituants identiques ou différents halogéno ou -CF₃, ou d'un de ses sels pharmaceutiquement acceptables.
